Europäisches Patentamt

European Patent Office

Office européen des brevets

(11) Publication number: **0 251 107**
A2

(12) **EUROPEAN PATENT APPLICATION**

(21) Application number: 87108921.5

(22) Date of filing: 22.06.87

(51) Int. Cl.⁴: **C12N 5/00** , C12N 15/00 , C12P 21/02 , C12P 33/00 , //(C12P21/02,C12R1:91),(C12P-33/00,C12R1:91)

(30) Priority: 24.06.86 US 878092

(43) Date of publication of application:
07.01.88 Bulletin 88/01

(84) Designated Contracting States:
AT BE CH DE ES FR GB GR IT LI LU NL SE

(71) Applicant: MERRELL DOW
PHARMACEUTICALS INC.
2110 E. Galbraith Road
Cincinnati Ohio 45215(US)

(72) Inventor: Sunkara, Sai P.
9629 Linfield Drive
Cincinatti Ohio 45242(US)

(74) Representative: Macchetta, Francesco et al
Gruppo Lepetit S.p.A. Patent and Trademark
Department 34, Via Roberto Lepetit
I-21040 Gerenzano (Varese)(IT)

(54) Improved fusion products.

(57) The present invention relates to a method for fusing non-antibody-producing mammalian cell with a mammalian cell capable of continuous propagation with high efficiency which includes agglutinating the cells prior to fusion. This method is also useful for fusing plant cells to give hybrid cells which are capable of producing useful products.

EP 0 251 107 A2

## IMPROVED FUSION PRODUCTS

The fusion of mouse myeloma cells to spleen cells from immunized mice by Kohler and Milstein in 1975 (nature 256 (1975), 495-497) demonstrated for the first time that it was possible to obtain a continuous cell line making homogeneous (so-called "monoclonal") and to the use of antibodies made by these hybridomas for various scientific investigations and diagnostic purposes in medicine. The method for preparing the hybridoma generally comprises the following steps:

(a) Immunizing e.g., mice or rats with an immunogenic agent. The immunization protocol should be such as to produce useful quantities of suitably primed splenocytes.

(b) Removing spleens from the immunized animal and making a spleen suspension in an appropriate medium.

(c) Fusing the suspended spleen cells with myeloma cells from a suitable cell line in the presence of a suitable fusion promoter. The fusion promoter could be sendai virus, a chemical fusogen, e.g., polyethylene glycol (PEG) having an average molecular weight of about 1000 to about 4000 (commercially available as PEG 1000, etc.). Another technique which is useful in effecting fusion is the electo-fusion according to the known methods such as those utilized by Zimmermann and Scheurich, Planta, 151 (1981), 26-32, Vienken et al., Planta, 157 (1983), 331, and Jacob et al., Sbud. Biophys., 94 (1983), 99. The myeloma cell line used should preferably be of the so-called "drug-resistant" type, so that unfused myeloma cells will not survive in a selective medium, while hybrids will. The most common class is 8-azaguanine resistant cell lines which lack the enzyme hypoxanthine guanine phosphoribosyl transferase and hence will not be supported by HAT (hypoxanthine, aminopterin, and thymidine) medium.

(d) Diluting and culturing the separate containers the mixture of unfused spleen cells, unfused myeloma cells and fused cells in the selecting medium which will not support the unfused myeloma cells for a time sufficient to allow death of unfused cells (about one week). The dilution maybe a type of limiting one, in which the volume of diluent is statistically calculated to isolate a certain number of cells (e.g., each well of a microtiter plate). In the selective medium the unfused myeloma cells perish. Since the unfused spleen cells are non-malignant they have only a finite number of generations. Thus, after a certain period of time (about one week) these unfused spleen cells fail to reproduce. The fused cells, on the other hand, continue to reproduce because they possess the malignant quality of the myeloma parent and the ability to survive in the selective medium of the spleen cell parent.

(e) Evaluating the supernatant in each container (well) containing a hybridoma for the presence of antibody directed against the antigene originally used.

(f) Selecting (e.g., by limiting dilution) and cloning hybridomas producing the desired antibody.

Once the desired hybridoma has been selected and cloned, the resultant antibody may be produced in one of two ways. The purest monoclonal antibody is produced by in vitro culturing of the desired hybridoma in a suitable medium for a suitable length of time, followed by recovery of the desired antibody from the supernatant. The suitable medium and suitable length of culturing time are known or are readily determined. This in vitro technique produces essentially monospecific monoclonal antibody, essentially free from other specific antihuman immune globulin. There is a small amount of other immune globulin present since the medium contains xenogeneic serum (e.g., fetal calf serum). However, this in vitro method may not produce a sufficient quantity or concentration of antibody for some purposes, since the concentration of monoclonal antibody is only about 50 μg/ml.

To produce a much greater concentration of slightly less pure monoclonal antibody, the desired hybridoma may be injected into mice, preferably syngenic or semi-syngenic mice. The hybridoma will cause formation of antibody-producing tumors after a suitable incubation time, which will result in a high concentration of the desired antibody (about 5-20 mg/ml) in the bloodstream and peritoneal exudate (ascites) of the host mouse.

This method is extensively disclosed in the literature. It has particular and serious drawbacks. PEG has gained increasing acceptance as a fusogen because it is available in pure form and in the desired molecular weight range, and because of its easy handling compared to the sendai virus. However, the range of concentrations over which PEG is effective is very narrow. The optimal concentration is 50 ± 5% weight/volume. At this concentration it exhibits significant cytotoxic effects against all cells to be fused and against the fused hybrid cells. When using suboptimal concentration of PEG, on the other hand, only a mode-rate cytotoxicity is observed, but the fusion efficiency and consequently the formation of hybridoma is low. In contrast to the sendai virus, the known chemical fusogens such as PEG and electro-fusion do not effect agglutination of the cells to be fused so that the cell membranes are not in close proximity efficient for fusion.

Thus, one object of the invention is to provide novel fused hybrid cells capable of producing a useful product. A further object of the invention is to develop a method for preparing a hybrid cell by fusing a non-antibody-producing mammalian cell or plant cell capable of producing a useful product, e.g., Langerhans islet cells, with a suitable fusion partner capable of continuous propagation. Another object of this invention is to reduce the cytotoxic effect of conventional chemical fusogens such as PEG, Lysolecithin, dextran, DMSO, polyvinyl alcohol, poly-L-ornithin and salts known to be useful such as sodium nitrate or a combination thereof. In the fusion process this results in a higher yield of the desired hybrid cells. Another object is to produce agglutinated cells to be fused by electro-fusion techniques.

These and other objects will become apparent from the following description. The present invention in its generic concept relates to a fused hybrid cell capable of producing a useful product obtained by the fusion of a non-antibody-producing mammalian cell with a mammalian cell capable of continuous propagation, said fusion having been facilitated by first causing agglutination and then effecting fusion of the agglutinated cells. In another aspect the invention in its generic concept relates to the agglutination and fusion of plant cells to produce hybrid cells capable of producing useful products. The present invention relates to a method for preparing a fused hybrid cell by fusing a non-antibody-producing mammalian cell capable of producing a useful product with a mammalian cell capable of continuous propagation under selective conditions which comprises contacting a mixture of cells to be fused with an effective amount of an agglutinogen and thereafter subjecting the agglutinated cells to fusion. In another aspect the invention relates to the agglutination and fusion of plant cells to produce hybrid cells capable of producing useful products. The present invention furthermore relates to a method for preparing a fused hybrid cell which comprises contacting a plant cell capable of producing a useful product and a plant cell capable of continuous propagation with an effective amount of an agglutinogen and thereafter subjecting the agglutinated cells to fusion.

Another aspect of this invention is the fusion of protoplasts from plant cells capable of producing useful products with protoplasts from cells capable of continuous propagation, (e.g., crown gall tumor cells) by known fusion techniques either with or without the benefit of prior or concommitant agglutination, these hybrid cells being capable of producing the desired useful product.

An "agglutinogen" is a term used to describe any agent capable of agglutinating the cells to be fused. Although all agglutinogens are embraced typical agglutinogens are phytohaemagglutinin (PHA), concanavallin A and peanut agglutinin. The preferred agglutinogen according to the present invention is PHA. The concentration of the agglutinogen should be adjusted such that no cytotoxic effects are observed and agglutination occurs to a significant degree.

A suitable range of the agglutinogen PHA is 25 - 400 $\mu$g/ml. The preferred concentration of PHA is about l50 - 200 $\mu$g/ml with 200 $\mu$g/ml being most preferred. The suitable concentration of the other agglutinogens may be readily determined by standard techniques well known in the art. Agglutination treatment is carried out at physiological temperatures, e.g., 37°C and for a sufficient length of time, e.g., 5 - l5 minutes. After sufficient agglutination has occured, the cells are subjected to fusion. In one embodiment a conventional chemical fusogen, preferably PEG is added and the mixture is incubated under physiological conditions to effect fusion of the agglutinated cells. When using PEG, the concentration is about 30 - 50% weight/volume. The preferred concentration of PEG is about 40 ± 5% weight/volume. The optimal fusion time is about l minute. Longer times can be used with the risk of cytotoxicity increasing with increased time, particularly above 2 minutes. In another embodiment fusion of the agglutinated cells is effected by electro-fusion.

This novel fusion technique of the present invention results in significantly increased viable hybrid cell formation compared to prior art results.

The term "useful product" embraces all products a mammalian cell or a plant cell to be fused may produce. Typical examples are biologically active proteins, glycoproteins, polypeptides, enzymes and non-protinaceous compounds such as alkaloids, steroids, diosgenin, anthraquinones, pyrethrins, essential oils, polysaccharides, cardiac glycosides, perfume and cosmetic components.

Typical examples of biologically active substances that can be produced from the cells to be fused are shown in the following tables I to V.

## TABLE I

| | |
|---|---|
| Alkaloids | Insecticides |
| Allergens | Latex |
| Anthraquinones | Lipids |
| Antileukaemic agents | Naphthoquinones |
| Antitumour agents | Nucleic acids |
| Antiviral agents | Nucleotides |
| Aromas | Oils |
| Benzoquinones | Opiates |
| Carbohydrates (including polysaccharides) | Organic acids |
| Cardiac glycosides | Peptides |
| Chalcones | Perfumes |
| Dianthrenes | Phenols |
| Enzymes | Pigments |
| Enzyme inhibitors | Plant growth regulators |
| Flavanoids, flavones | Proteins |
| Flavours (including sweeteners) | Steroids and derivatives |
| Fluranocoumarins | Sugars |
| Hormones | Tannis |
| | Terpenes and terpenoids |
| | Vitamins |

TABLE II

| Biologically Active Substance | Cell Source | Use |
| --- | --- | --- |
| Insulin | Pancreatic islet cells | Antidiabetic |
| Glucagon | Pancreatic islet cells | Regulates the insulin production |
| Somatostatin | Pancreatic islet cells | Regulates the insulin production |
| ACTH | Anterior pituitary cells | Anti-inflammatory |
| Lutenizing hormone | Pituitary cells | Regulate reproduction |
| Follicle stimulating hormone | Pituitary cells | Regulate reproduciton |
| Growth hormone | Pituitary cells | Promotes growth |
| Lutenizing Hormone-Release Hormone | Pituitary cells | Regulates fertility |
| Prolactin | Pituitary cells | Stimulates milk production |
| Thyrotropin (TSH) | Pituitary cells | Hypothyrodism |
| Thymic hormones | Thymus cells | Immunomodulator |
| Erythropoietin | Kidney | Stimulates erythropoiesis |
| Epidermal growth factor | Submaxillary gland | Promotes cells proliferation and inhibits gastric acid secretion |
| Oxytocin | Posterior pituitary | Controls uterine contraction and milk production |
| Vasopressin | Posterior pituitary | Antidiuretic hormone |
| Enkephalin | Adrenal chromaffin cells | Analgesic |

0 251 107

TABLE II (Continued)

| | | |
|---|---|---|
| (ANF) Atrial Naturietic Factor | Heart cells | Vasodialator |
| $\beta$-endorphins | Brain, pituitary gland | Analgesic |
| Inhibin | Granulosa cells (ovary) | Fertility control |
| Calcitonin | Thyroid cells | Increases deposition of $Ca^{++}$ in bones |
| Parathyroid hormone | Parathyroid (principal cell) | Causes resorption of bone |
| Interleukin-1 | Macrophage | Antitumor |
| Interleukin-2 | T helper cell | Antitumor |
| Interleukin-3 | T helper cell | Stem cell proliferation |
| $\alpha$-Interferon | Leukocytes | Antiviral, antitumor |
| Colony stimulating factor-2 | T cell | MQ and granulocyte proliferation |
| CSF-1 | Fibroblasts | MQ growth and activation |
| B cell growth factor | T helper cell | Immunodefficiency |
| Tumor necrosis factor | Macrophage | Antitumor |
| $\gamma$-Interferon | Macrophage, T helper cells | Antiviral and antitumor |
| $\beta$-Interferon | Fibroblast | Antiviral and antitumor |
| Macrophage activating factor | Macrophage | Antitumor and antiviral |
| Angiogenin | Carcinoma cells | Wound healer, vascularlization |
| Steroids | Adrenal cortica | Reproduction |
| Nerve growth factor | Salivary gland cells | Neuronal regeneration |
| Platlet derived growth factor | (Endothine platlet) | Wound healing |
| Melanophore stimulating hormone and melatonin | Pinearocyte | Pigmentation control |

0 251 107

TABLE III

| Compound group | Type and examples |
|---|---|
| Pharmaceuticals | Alkaloids, steroids, anthraquinones |
| Enzymes | Proteases (e.g., papain) |
| Latex | Isoprenoids (e.g., rubber) |
| Waxes | Wax esters (e.g., jojoba) |
| Pigments | Stains and dyes |
| Oils | Fatty acids (e.g., seed oils) |
| Agrochemicals | Insecticides (e.g., pyrethrins) |
| Cosmetic substances | Essential oils (e.g., monoterpenes) |
| Food additives | Flavour compounds, non-nutritive sweeteners (e.g., thaumatin) |
| Gums | Polysaccharides (e.g., gum arabic) |

TABLE IV

| Industry | Plant product | Plant species | Industrial uses |
|---|---|---|---|
| Pharmaceuticals | Codeine (alkaloid) | Papaver somniferum | Analgesic |
| | Diogenin (steroid) | Dioscorea deltoidia | Anti-fertility agents |
| | Quinine (alkaloid) | Cinchona ledgeriana | Antimalarial |
| | Digoxin (caridac glycoside) | Digitalis lanata | Cardiatonic |
| | Scopolamine (alkaloid) | Datura stramonium | Antihypertensive |
| | Vincristine (alkaloid) | Catharanthus roseus | Antileukaemic |
| Agrochemicals | Pyrethrin | Chrysanthemum cinerariaefolium | Insecticide |
| Food and drink | Quinine (alkaloid) | Cinchona ledgeriana | Bittering agent |
| | Thaumatin (chalcone) | Thaumatococcus danielli | Non-nutritive sweetener |
| Cosmetics | Jasmine | Jasminum sp. | Perfume |

TABLE V

| Medicinal agent | Activity | Plant source |
|---|---|---|
| Steroids from diosgenin | Anti-fertility agents | Dioscorea deltiodea |
| Codeine | Analgesic | Papaver somniferum |
| Atropine | Anticholinergic | Atropa belladonna |
| Reserpine | Antihypertensive | Rauwolfia serpentina |
| Hyoscyamine | Anticholinergic | Hyoscyamus niger |
| Digoxin | Cardiatonic | Digitalis lanata |
| Scopolamine | Anticholinergic | Datura metel |
| Digitoxin | Cardiovascular | Digitalis purpurea |
| Pilocarpine | Cholinergic | Pilocarpus jabonandi |
| Quinidine | Antimalarial | Cinchona ledgeriana |

Non-antibody-producing cells of mammalian origin and plant cells may be used, plant cells preferably in the form of protoplasts. Protoplast formation is well known by those of ordinary skill in the art.

Furthermore, mammalian cells may be used which produce biologically active substances other than proteins such as steroid hormones. In the case of plant cells, cells may be used which produce alkaloids such as quinine, reserpine, cocaine, atropine, scopolamine, digitalis, morphine-like substances.

Furthermore, cells may be used which produce essential oils and scents such as muscone, civetone, geraniol and other terpene-like substances useful in the perfume industry.

Examples of continuously propagating (permanent) cells to be used as fusion partners and for the immortalization of mammalian and plant cells are myeloma cells of various origin such as mouse, rat or human origin. Typical examples of permanently growing cells are tumor cells such as myeloma cells, cells derived from human neoplasia including HeLa cells, HE p-2 derived from pharynx cancer cells and KB derived from rhino-pharyngo cancer cells, lymphoblastoid cells, Epstein-Barr virus transformed cells, highly propagating embryo cells, hepatoma cells, renal carcinoma cells. Of particular importance for the present invention are drug resistant myelomoa cells, including murine and human myeloma cells. it is also generally preferred that the myeloma cells used be of the so-called "non-secreting" type, although secreting types may be used.

Typically continuously growing plant cells are tumorous crown gall cells, i.e., plant cells transformed by a virulent strain of Agrobacterium tumefaciens and protoplasts thereof.

Similarly, it is also contemplated that the generic aspects of this invention embrace the formation of hybrid cells obtained by fusing any prokaroyte or eukaryote type cells (capable of producing a useful substance) with any appropriate immortalizing cell, whether or not the immortalizing cell is a mammalian or plant cell.

The fused cells are grown or propagated in conventional nutrient media containing conventional sources of carbon, nitrogen and mineral salts. The propagation is carried out generally under aerobic conditions, i.e., in the presence of a mixture of 95% oxygen and 5% carbon dioxide. It is evident that propagation and all preceding steps are carried out under sterile conditions. Larger amounts of hybrid cells may be produced in syngenic or athymic nude mice according to conventional methods. The cells can be harvested in the form of ascites or solid tumors and propagated as conventional cell cultures. Such cell cultures can be induced to produce larger amounts of the desired products. A typical example is the induction of hybrid cells derived from Langerhans islet cells induced with glucose to form increased amounts of insulin. In an analogous manner, the production of urokinase can be stimulated by adding glycine to urokinase producing hybrid cells.

It is a particularly important and novel feature of this invention that the process facilitates the production of a variety of hybrid cells, each of which is capable of producing their own particular useful product, in the same medium. For example (as shown in Example I), the fusion of islet cells obtained from the pancreas, (said cell mixture containing A, B, C and D type cells) with an immortalizing cell is capable of producing a mixture of hybrid cells, some of which will produce glucagon, some of which will produce somatostatin, some of which will produce insulin, etc.

The isolation of the useful product produced by the hybrid cells is carried out in any conventional manner, e.g., by removing the nutrient medium from the hybrid cells and by extraction, counter-current distribution, affinity chromatography, precipitation, gel filtration, ion exchange chromatography, HPLC etc., and a combination of these methods.

The products are in general well known and identified and their use therefore is also well known.

The invention is explained in detail in the following examples:

## EXAMPLE I

Remove pancreatic tissue from new born mice (one week old) and collect into 10 ml of sterile Hank solution in a 100 mm petri dish. The pancreas tissue is freed from connective tissue and fat and minced into small (1 $\times$ 1 mm) pieces and washed twice with sterile saline solution before digestion with collagenase and trypsin. Transfer the pancreatic tissue into a small flask (25 ml) and add 10 ml of trypsin (2.5% in saline, Gibco) and 2 ml of collagenase (3 mg/ml Sigma). Incubate for 15 min. with gentle magnetic stirring at 37°C and discard the supernatant so-obtained. Repeat the trypsin-collagenase treatment 3 times and save the supernatants obtained each time at 4 C. Suspend the cell pellets obtained by centrifugation (600 g) in 30 ml of MEM medium with 16.7 mM glucose in a 100 mm dish and incubate at 37°C with 95% oxygen and 5% $CO_2$ for 22 hrs. Collect the unattached floating islet cells, centrifuge and wash once with Hanks solution. Mix the islet cells so obtained (6 $\times$ 10$^7$ cells) with mouse myeloma (FOX - NY) cells (6

× 10⁶) in a sterile tube (l5 ml Corning). Wash the cell mixture once with Hank solution by centrifugation. Suspend the pellet so obtained gently and add 0.2 ml of PHA (200 ug/ml) and incubate the tube for lO min. at 37°C. At the end of incubation add to the tube 0.8 ml of PEG 50% weight/volume (average molecular weight l000) solution to obtain a final concentration of 40% PEG. After I min. incubation at 37°C, to the tube add l0 ml of the MEM medium and incubate for I hr. at 37°C. Wash the cells by centrifugation (600 g) and suspend in 20 ml of HAT medium and dispense 0.2 ml/well into 96 well plates (Corning). Incubate the plates at 37°C in a $CO_2$ incubator. Change the medium after a week and at the end of 2 weeks assay the wells for the presence of insulin by radioimmuno assay (RIA). Subculture and maintain the cells from positive wells. This procedure yields 24 positive wells producing 80 - 200 micro units of insulin after 48 hrs. of cultivation.

In this same experiment, the wells were also assayed for somatostatin and glulcagon by radioimmuno assay (RIA) techniques. Subculture and maintainence of the cells from positive wells showed that 2l wells contained hybrid cells producing 300-400 *f*-moles/ml of glucagon after 48 hours of cultivation were prepared by this example and that 3 wells contained hybrid cells producing approximately l00 - 500 *f*-moles/ml of somatostatin after 48 hours of cultivation.

## EXAMPLE 2

Activated peritoneal macrophages obtained from Corynebacterium parvum treated mice are fused with mouse myeloma cells according to the procedure of Example I. The hybrid cells obtained are screened for interleukin I, gamma interferon, tumor necrosis factor and macrophage activating factor. This produced six wells of positive for gamma interferon. Gamma interferon produced was approximately l00 - 300 units per ml.

## ˙EXAMPLE 3

T-lymphocytes obtained from mouse spleens are activated by mixed lymphocyte reaction and then fused as described in Example I. The obtained cell hybrids are screened for interleukin 2, 3 and B cell growth factor.

## EXAMPLE 4

Cells from the pituitary gland are fused as described in Example I and the obtained cell hybrids are screened for growth hormones and prolactin utilizing hormones.

## EXAMPLE 5

Protoplasts from crown gall tumors (induced by a virulent strain of Agrobacterium tumefaciens of a tobacco plant) are fused with protoplasts obtained from Rauwolfia serpentina in accordance with the procedure of Example I and the rapidly dividing hybrid cells are extracted by established procedures to obtain reserpine.

## EXAMPLE 6

Protoplasts from crown gall tumors of a tobacco plant are fused with protoplasts obtained from Digitalis purpurea in accordance with the procedure of Example I and the so-obtained hybrid cells are extracted to obtain a mixture of digitalis glycosides.

## EXAMPLE 7

Protoplasts of crown gall tumors of a tobacco plant are fused with protoplasts obtained from Atropa belladonna in accordance with the procedure of Example I and the so-obtained hybrid cells are extracted to obtain atropine.

**Claims**

I. A fused hybrid cell capable of producing a useful product obtained by the fusion of a non-antibody-producing mammalian cell with a mammalian cell capable of continuous propagation, said fusion having been facilitated by first causing agglutination and then effecting fusion of the agglutinated cells.

2. A fused hybrid cell capable of producing a useful product obtained by the fusion of a plant cell with a plant cell capable of continuous propagation, said fusion having been facilitated by first causing agglutination and then effecting fusion of the agglutinated cells.

3. The useful product produced by the hybrid cell of Claim I or 2.

4. A method for preparing a hybrid cell by fusing a non-antibody-producing mammalian cell with a mammalian cell capable of continuous propagation under fusion and under selective conditions which comprises contacting the mixture of cells to be fused with an effective amount of an agglutinogen and thereafter effecting fusion of the agglutinated cells.

5. A method for preparing a fused hybrid cell which comprises contacting a plant cell capable of producing a useful product and a plant cell capable of continuous propagation with an effective amount of an agglutinogen and thereafter effecting fusion of the agglultinated cells.

6. A method according to Claim 4 or 5, characterized in that the agglutinogen is phytohaemagglutinin.

7. A method according to Claim 4 or 5, characterized in that the fusion is effected by chemical fusion.

8. A method according to Claim 4 or 5, characterized in that the fusion is effected by electrofusion.

9. A method according to Claim 7, characterized in that the fusogen is PEG having an average molecular weight from about 1000 to about 4000.

10. A method according to Claim 5, characterized in that the plant cell capable of continuous propagation is a tumorous crown gall cell.

11. A method according to Claim 1 wherein the continuously propagating cell is a myeloma.

12. A fused hybrid cell of Claim 1 capable of producing insulin, said cell being a fusion product of a pancreatic islet cell and a myeloma cell.

13. A fused hybrid cell of Claim 1 capable of producing glucagon, said cell being a fusion product of a pancreatic islet cell and a myeloma cell.

14. A fused hybrid cell of Claim 1 capable of producing somatostatin, said cell being a fusion product of a pancreatic islet cell and a myeloma cell.

15. A fused hybrid cell of Claim 1 capable of producing ACTH, said cell being a fusion product of an anterior pituitary cell and a myeloma cell.

16. A fused hybrid cell of Claim 1 capable of producing lutenizing hormone, said cell being a fusion product of a pituitary cell and a myeloma cell.

17. A fused hybrid cell of Claim 1 capable of producing follicle stimulating hormone, said cell being a fusion product of a pituitary cell and a myeloma cell.

18. A fused hybrid cell of Claim 1 capable of producing growth hormone, said cell being a fusion product of a pituitary cell and a myeloma cell.

19. A fused hybrid cell of Claim 1 capable of producing LH-RH, said cell being a fusion product of a pituitary cell and a myeloma cell.

20. A fused hybrid cell of Claim 1 capable of producing prolactin, said cell being a fusion product of a pituitary cell and myeloma cell.

21. A fused hybrid cell of Claim 1 capable of producing thyrotropin (TSH), said cell being a fusion product of a pituitary cell and a myeloma cell.

22. A fused hybrid cell of Claim 1 capable of producing thymic hormones, said cell being a fusion product of a thymus cell and a myeloma cell.

23. A fused hybrid cell of Claim 1 capable of producing erythropoietin, said cell being a fusion product of a kidney cell and a myeloma cell.

24. A fused hybrid cell of Claim 1 capable of producing epidermal growth factor, said cell being a fusion product of a submaxillary gland cell and a myeloma cell.

25. A fused hybrid cell of Claim 1 capable of producing oxytocin, said cell being a fusion product of a posterior pituitary cell and a myeloma cell.

26. A fused hybrid cell of Claim 1 capable of producing vasopressin, said cell being a fusion product of a posterior pituitary cell and a myeloma cell.

27. A fused hybrid cell of Claim 1 capable of producing enkephalin, said cell being a fusion product of an adrenal chromaffin cell and a myeloma cell.

28. A fused hybrid cell of Claim 1 capable of producing atriol nutrivite factor (ANF), said cell being a fusion product of a heart cell and a myeloma cell.

29. A fused hybrid cell of Claim 1 capable of producing endorphins, said cell being a fusion product of a brain or a pituitary gland cell and a myeloma cell.

30. A fused hybrid cell of Claim 1 capable of producing inhibin, said cell being a fusion product of a granulosa cell (ovary) and a myeloma cell.

31. A fused hybrid cell of Claim 1 capable of producing calcitonin, said cell being a fusion product of a thyroid cell and a myeloma cell.

32. A fused hybrid cell of Claim 1 capable of producing parathyroid hormone, said cell being a fusion product of a parathyroid cell and a myeloma cell.

33. A fused hybrid cell of Claim 1 capable of producing Interleukin-1, said cell being a fusion product of a macrophage cell and a myeloma cell.

34. A fused hybrid cell of Claiim 1 capable of producing Interleukin-2, said cell being a fusion product of a T helper cell and a myeloma cell.

35. A fused hybrid cell of Claim 1 capable of producing Interleukin-3, said cell being a fusion product of a T helper cell and a myeloma cell.

36. A fused hybrid cell of Claim 1 capable of producing α-Interferon, said cell being a fusion product of leukocytes and a myeloma cell.

37. A fused hybrid cell of Claim 1 capable of producing colony stimulating factor-2, said cell being a fusion product of a T cell and a myeloma cell.

38. A fused hybrid cell of Claim I capable of producing CSF-I, said cell being a fusion product of fibroblast and a myeloma cell.

39. A fused hybrid cell of Claim I capable of producing B cell growth factor, said cell being a fusion product of a T helper cell and a myeloma cell.

40. A fused hybrid cell of Claim I capable of producing tumor necrosis factor, said cell being a fusion product of a macrophage cell and a myeloma cell.

41. A fused hybrid cell of Claim I capable of producing $\gamma$-Interferon, said cell being a fusion product of a macrophage cell or T helper cell and a myeloma cell.

42. A fused hybrid cell of Claim I capable of producing $\beta$-Interferon, said cell being a fusion product of a fibroblast cell and a myeloma cell.

43. A fused hybrid cell of Claim I capable of producing macrophage activating factor, said cell being a fusion product of a macrophage cell and a myeloma cell.

44. A fused hybrid cell of Claim I capable of producing angiogenin, said cell being a fusion product of carcinoma cells and a myeloma cell.

45. A fused hybrid cell of Claim I capable of producing steroids, said cell being a fusion product of adrenal cortica and a myeloma cell.

46. A fused hybrid cell of Claim I capable of producing nerve growth factor, said cell being a fusion product of salivary gland cells and a myeloma cell.

47. A fused hybrid cell of Claim I capable of producinig platlet derived GF, said cell being a fusion product of endothine platlets and a myeloma cell.

48. A fused hybrid cell of Claim I capable of producing MSH and melatonin, said cell being a fusion product of pinealcyte and a myeloma cell.

Claims for the following contracting states: AT, ES, GR.

1. A method for preparing a hybrid cell by fusing a non-antibody-producing mammalian cell with a mammalian cell capable of continuous propagation under fusion and under selective conditions which comprises contacting the mixture of cells to be fused with an effective amount of an agglutinogen and thereafter effecting fusion of the agglutinated cells.

2. A method for preparing a fused hybrid cell which comprises contacting a plant cell capable of producing a useful product and a plant cell capable of continuous propagation with an effective amount of an agglutinogen and thereafter effecting fusion of the agglutinated cells.

3. A method according to Claim 1 or 2 wherein the agglutinogen is phytohaemagglutinin.

4. A method according to Claim 1 or 2, wherein the fusion is effected by chemical fusion.

5. A method according to Claim 1 or 2, wherein the fusion is effected by electro-fusion.

6. A method according to Claim 4, wherein the fusogen is PEG having an average molecular weight from about 1000 to about 4000.

7. A method according to Claim 2, wherein the plant cell capable of continuous propagation is a tumorous crown gall cell.

8. A method according to Claim 1, wherein the continuously propagating cell is a myeloma.

9. A method according to Claim 1 for preparing a fused hybrid cell capable of producing insulin, said cell being a fusion product of a pancreatic islet cell and a myeloma cell.

10. A method according to Claim 1 for preparing a fused hybrid cell capable of producing glucagon, said cell being a fusion product of a pancreatic islet cell and a myeloma cell.

11. A method according to Claim 1 for preparing a fused hybrid cell capable of producing somatostatin, said cell being a fusion product of a pancreatic islet cell and a myeloma cell.

12. A method according to Claim 1 for preparing a fused hybrid cell capable of producing ACTH, said cell being a fusion product of an anterior pituitary cell and a myeloma cell.

13. A method according to Claim 1 for preparing a fused hybrid cell capable of producing luteinizing hormone, said cell being a fusion product of a pituitary cell and a myeloma cell.

14. A method according to Claim 1 for preparing a fused hybrid cell capable of producing follicle stimulating hormone, said cell being a fusion product of a pituitary cell and a myeloma cell.

15. A method according to Claim 1 for preparing a fused hybrid cell capable of producing growth hormone, said cell being a fusion product of a pituitary cell and a myeloma cell.

16. A method according to Claim 1 for preparing a fused hybrid cell capable of producing LH-RH, said cell being a fusion product of a pituitary cell and a myeloma cell.

17. A method according to Claim 1 for preparing a fused hybrid cell capable of producing prolactin, said cell being a fusion product of a pituitary cell and myeloma cell.

18. A method according to Claim 1 for preparing a fused hybrid cell capable of producing thyrotropin (TSH), said cell being a fusion product of a pituitary cell and a myeloma cell.

19. A method according to Claim 1 for preparing a fused hybrid cell capable of producing thymic hormones, said cell being a fusion product of a thymus cell and a myeloma cell.

20. A method according to Claim 1 for preparing a fused hybrid cell capable of producing erythropoietin, said cell being a fusion product of a kidney cell and a myeloma cell.

21. A method according to Claim 1 for preparing a fused hybrid cell capable of producing epidermal growth factor, said cell being a fusion product of a submaxillary gland cell and a myeloma cell.

22. A method according to Claim 1 for preparing a fused hybrid cell capable of producing oxytocin, said cell being a fusion product of a posterior pituitary cell and a myeloma cell.

23. A method according to Claim 1 for preparing a fused hybrid cell capable of producing vasopressin, said cell being a fusion product of a posterior pituitary cell and a myeloma cell.

24. A method according to Claim 1 for preparing a fused hybrid cell capable of producing enkephalin, said cell being a fusion product of an adrenal chromaffin cell and a myeloma cell.

25. A method according to Claim 1 for preparing a fused hybrid cell capable of producing atrial natriuretic factor (ANF), said cell being a fusion product of a heart cell and a myeloma cell.

26. A method according to Claim 1 for preparing a fused hybrid cell capable of producing endorphins, said cell being a fusion product of a brain or a pituitary gland cell and a myeloma cell.

27. A method according to Claim 1 for preparing a fused hybrid cell capable of producing inhibin, said cell being a fusion product of a granulosa cell (ovary) and a myeloma cell.

28. A method according to Claim 1 for preparing a fused hybrid cell capable of producing calcitonin, said cell being a fusion product of a thyroid cell and a myeloma cell.

29. A method according to Claim 1 for preparing a fused hybrid cell capable of producing parathyroid hormone, said cell being a fusion product of a parathyroid cell and a myeloma cell.

30. A method according to Claim 1 for preparing a fused hybrid cell capable of producing Interleukin-1, said cell being a fusion product of a macrophage cell and a myeloma cell.

31. A method according to Claim 1 for preparing a fused hybrid cell capable of producing Interleukin-2, said cell being a fusion product of a T helper cell and a myeloma cell.

32. A method according to Claim 1 for preparing a fused hybrid cell capable of producing Interleukin-3, said cell being a fusion product of a T helper cell and a myeloma cell.

33. A method according to Claim 1 for preparing a fused hybrid cell capable of producing $\alpha$-Interferon, said cell being a fusion product of leukocytes and a myeloma cell.

34. A method according to Claim 1 for preparing a fused hybrid cell capable of producing colony stimulating factor-2, said cell being a fusion product of a T cell and a myeloma cell.

35. A method according to Claim 1 for preparing a fused hybrid cell capable of producing CSF-1, said cell being a fusion product of fibroblast and a myeloma cell.

36. A method according to Claim 1 for preparing a fused hybrid cell capable of producing B cell growth factor, said cell being a fusion product of a T helper cell and a myeloma cell.

37. A method according to Claim 1 for preparing a fused hybrid cell capable of producing tumor necrosis factor, said cell being a fusion product of a macrophage cell and a myeloma cell.

38. A method according to Claim 1 for preparing a fused hybrid cell capable of producing $\gamma$-Interferon, said cell being a fusion product of a macrophage cell or T helper cell and a myeloma cell.

39. A method according to Claim 1 for preparing a fused hybrid cell capable of producing $\beta$-Interferon, said cell being a fusion product of a fibroblast cell and a myeloma cell.

40. A method according to Claim 1 for preparing a fused hybrid cell capable of producing macrophage activating factor, said cell being a fusion product of a macrophage cell and a myeloma cell.

41. A method according to Claim 1 for preparing a fused hybrid cell capable of producing angiogenin, said cell being a fusion product of carcinoma cells and a myeloma cell.

42. A method according to Claim 1 for preparing a fused hybrid cell capable of producing steroids, said cell being a fusion product of an adrenal cortical cell and a myeloma cell.

43. A method according to Claim 1 for preparing a fused hybrid cell capable of producing nerve growth factor, said cell being a fusion product of salivary gland cells and a myeloma cell.

44. A method according to Claim 1 for preparing a fused hybrid cell capable of producing platlet derived GF, said cell being a fusion product of endothine platlets and a myeloma cell.

45. A method according to Claim 1 for preparing a fused hybrid cell capable of producing MSH and melatonin, said cell being a fusion product of pinealcyte and a myeloma cell.